# EUROPÄISCHE PATENTANMELDUNG

(11) **EP 0 610 775 A1**
(43) Veröffentlichungstag der Anmeldung: **17.08.1994**
(21) Anmeldenummer: 94101442.5
(22) Anmeldetag: 01.02.1994
(51) Int. Cl.: B01D 61/36, B01D 3/36

(54) **Verfahren zur Trennung von Flüssigkeitsgemischen mit azeotropem Siedeverhalten durch Verschaltung von Pervaporation und Destillation**

(30) Priorität: 06.02.1993 DE 4303528
(71) Anmelder: BASF Aktiengesellschaft, D-67063 Ludwigshafen (DE)
(72) Erfinder: Dams, Albrecht, Dr., D-67157 Wachenheim (DE); Krug, Joseph, Dr., D-67273 Weisenheim (DE); Palm, Christof, Dr., D-67069 Ludwigshafen (DE)

(57) **Zusammenfassung**

Verfahren zur Trennung von Flüssigkeitsgemischen mit azeotropem Siedeverhalten durch Verschaltung von Pervaporation und Destillation, wobei
- in einem ersten Verfahrensschritt durch Pervaporation ein Gemisch soweit aufkonzentriert wird, daß die azeotrope Zusammensetzung überschritten wird, und
- in einem zweiten Verfahrensschritt die angereicherte Komponente bis zur geforderten Reinheit mittels einer Destillationseinheit aufdestilliert wird, und das dabei anfallende leichter oder schwerer siedende Gemisch der Pervaporation wieder zugeführt wird.

## Beschreibung

Die Erfindung betrifft ein Verfahren zur Trennung von Flüssigkeitsgemischen mit azeotropem Siedeverhalten durch Verschaltung von Pervaporation und Destillation.

Für die Trennung von Flüssigkeitsgemischen mit azeotropem Siedeverhalten werden in der Technik oft destillative Sonderverfahren eingesetzt. Bei diesen Verfahren wird entweder durch die Ausnutzung systemimmanenter Eigenschaften (Druckabhängigkeit des Azeotrops, Azeotrop innerhalb einer Mischungslücke) oder durch die Zugabe eines Hilfsstoffes die Destillationsgrenze überwunden. Diese Verfahren sind in Ullmanns Encyklopädie der technischen Chemie, 4. Auflage, Band 2, Seiten 489 bis 545, beschrieben.

Meist sind diese destillativen Sonderverfahren verfahrenstechnisch aufwendig, da sie mindestens zwei, häufig aber mehr Kolonnen benötigen sowie weitere Apparate, wie z.B. Druckerhöhungsstufen oder Dekanter. Weitere Nachteile bestehen im Energiebedarf dieser Verfahren sowie im Einsatz von Hilfsstoffen, beispielsweise bei der Schleppmitteldestillation.

Bekannt ist, daß Flüssigkeitsgemische mit azeotropem Siedeverhalten durch Pervaporation getrennt werden können.

Verfahren zur Trennung azeotroper Gemische durch Pervaporation sind beschrieben in J. of Membrane Science, 51 (1990), Seiten 169 bis 179, "Pervaporation of liquid mixtures through polyvinylalcohol (PVA) membranes" und AIChE Journal, 37 (1991) 4, Seiten 581 bis 588, "Separation of azeotropic organic liquid mixtures by pervaporation".

Bei der Pervaporation werden eine oder mehrere Komponenten durch eine für sie selektive Membran aus dem Gemisch entfernt und im Permeat angereichert. Dabei wird ein treibendes Gefälle durch Absenken der Partialdrücke der permeierenden Komponenten auf der Permeatseite erzeugt. Die nicht über die Membran abgetrennten Komponenten werden im Retentat aufkonzentriert.

Durch Pervaporation können Gemische in einem Verfahrensschritt in ihre Komponenten zerlegt werden, ohne daß die Trennung durch Azeotrope limitiert ist. Im Gegensatz zu destillativen Sonderverfahren erfordert die Pervaporation keine zusätzlichen Hilfsstoffe und ist oftmals energetisch günstiger.

Das Pervaporationsverfahren weist jedoch zwei verfahrenstechnische Nachteile auf:
- Die durch die Membrantrennung erreichbaren Reinheiten sind in der Regel durch den Unterdruck auf der Permeatseite limitiert. Z.B. liegt für die Entwässerung organischer Gemische der technisch erreichbare Wert bei 0,05 bis 0,1 Gew.-% Wasser im Retentat.
- Nebenkomponenten, für die die Membran nicht selektiv ist, verbleiben im Retentat und können nicht abgetrennt werden.

Der vorliegenden Erfindung liegt daher die Aufgabe zugrunde, die Pervaporation in einem Verfahren zur Trennung von Gemischen mit azeotropem Siedeverhalten so einzubinden, daß die verfahrenstechnischen Vorteile der Membrantrennung zur Geltung kommen, die oben genannten Nachteile der Pervaporation aber beseitigt werden.

Obige Aufgabe wird erfindungsgemäß dadurch gelöst, daß
- in einem ersten Verfahrensschritt durch Pervaporation ein Gemisch soweit aufkonzentriert wird, daß die azeotrope Zusammensetzung überschritten wird, und
- in einem zweiten Verfahrensschritt die angereicherte Komponente bis zur geforderten Reinheit mittels einer Destillationseinheit aufdestilliert wird, und das dabei anfallende leichter oder schwerer siedende Gemisch der Pervaporation wieder zugeführt wird.

Weitere Merkmale des erfindungsgemäßen Verfahrens sind Gegenstand der Unteransprüche.

Verschiedene Ausführungsbeispiele der Erfindung mit den wesentlichen erfinderischen Merkmalen sind in der Zeichnung dargestellt und werden im folgenden näher beschrieben.

In Figur 1 ist das Verfahren für die kontinuierliche Trennung eines Gemisches mit Minimumazeotrop und
in Figur 2 ist das Verfahren für die kontinuierliche Trennung eines Gemisches mit Maximumazeotrop dargestellt.

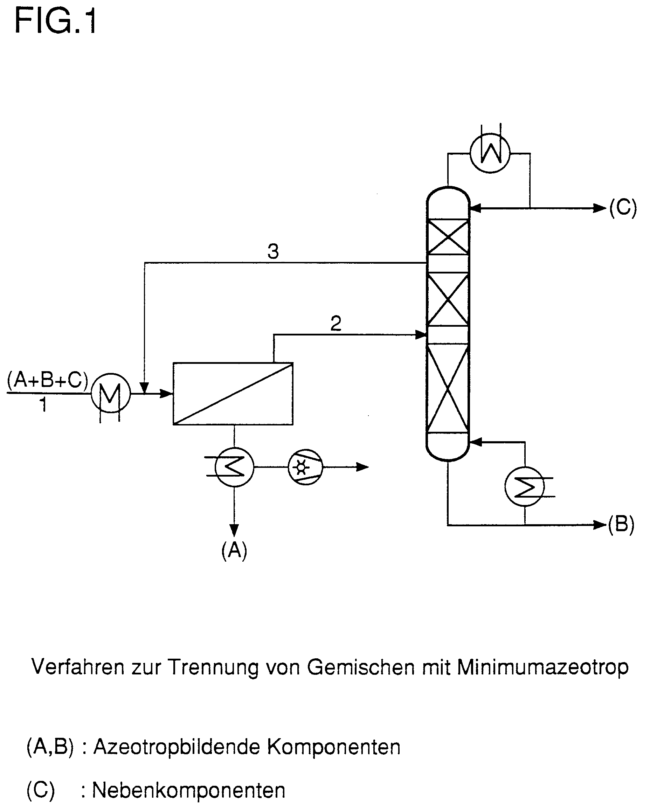

Figur 1, Trennung eines Gemisches mit Minimumazeotrop:
Durch die Pervaporation wird die Komponente (A) aus dem Flüssigkeitsgemisch (A, B, C) (Strom 1) soweit abgereichert, daß die azeotrope Zusammensetzung überwunden wird. Zur weiteren Abtrennung von (A) aus Strom 2 wird in der anschließenden Destillation ein Gemisch aus (A) und (B), z.B. mit azeotroper Zusammensetzung, aufdestilliert und zurückgeführt (Strom 3). Dadurch kann die Komponente (B) in reiner Form als Sumpfabzug gewonnen werden. Nebenkomponenten (C), die leichter flüchtig sind als das Azeotrop aus (A) und (B), können im selben Verfahrensschritt destillativ abgetrennt werden. In diesem Fall wird Strom 3 nicht als Kopfprodukt sondern als Seitenabzug im Verstärkungsteil entnommen. Die Destillationskolonne kann bei Normaldruck, erhöhtem Druck oder im Vakuum betrieben werden, um die azeotrope Zusammensetzung so zu verschieben, daß der rückgeführte Mengenstrom (Strom 3) und damit der Energieaufwand reduziert wird. Durch Optimierung des Drucks in der Destillationskolonne wird es weiterhin ermöglicht, die Kondensationswärme aus der Destillation für die Pervaporation im Wärmeverbund zu nutzen.

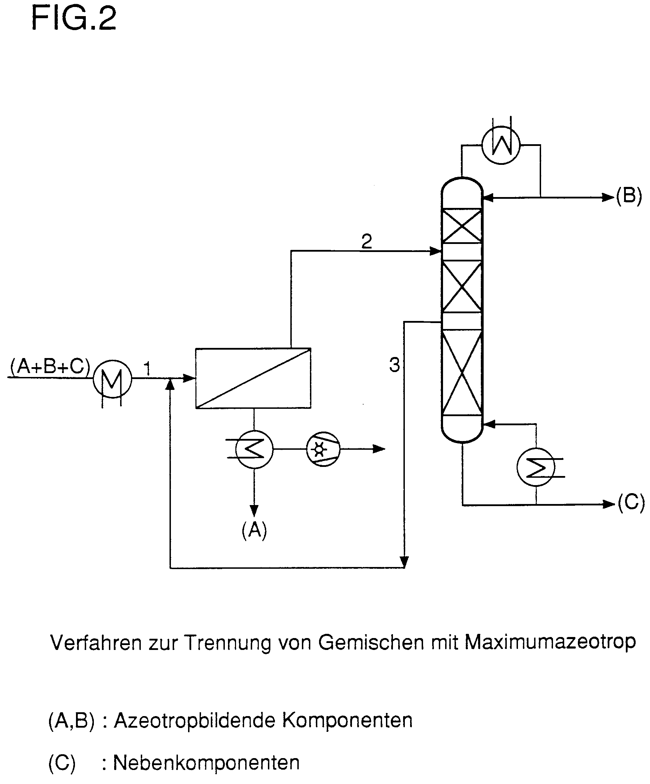

Figur 2, Trennung eines Gemisches mit Maximumazeotrop:
Durch die Pervaporation wird die Komponente (A) aus dem Flüssigkeitsgemisch (A, B, C) (Strom 1) soweit abgereichert, daß die azeotrope Zusammensetzung überwunden wird. Zur weiteren Abtrennung von (A) aus Strom 2 wird in der anschließenden Destillation ein Gemisch aus (A) und (B), z.B. mit azeotroper Zusammensetzung, aufdestilliert und zurückgeführt (Strom 3). Dadurch kann die Komponente (B) in reiner Form als Kopfabzug gewonnen werden. Nebenkomponenten (C), die schwerer flüchtig sind als das Azeotrop aus (A) und (B), können im selben Verfahrensschritt destillativ abgetrennt werden. In diesem Fall wird Strom 3 nicht als Sumpfprodukt sondern als Seitenabzug im Abtriebsteil entnommen. Die Destillationskolonne kann bei Normaldruck, erhöhtem Druck oder im Vakuum betrieben werden, um die azeotrope Zusammensetzung so zu verschieben, daß der rückgeführte Mengenstrom (Strom 3) und damit der Energieaufwand reduziert wird. Durch Optimierung des Drucks in der Destillationskolonne wird es weiterhin ermöglicht, die Kondensationswärme aus der Destillation für die Pervaporation im Wärmeverbund zu nutzen.

Folgende verfahrenstechnische Vorteile werden mit der Erfindung erzielt:
1. Die Trennsequenz ist einfacher und der Energiebedarf ist geringer als bei destillativen Sonderverfahren.
2. Es lassen sich höhere Reinheiten der Komponente (B) erzielen als bei einer Trennung nur durch Pervaporation.
3. Es können zusätzliche Nebenkomponenten, die nicht durch die Membran permeieren, abgetrennt werden.
4. Durch Wärmeverbund kann die Kondensationswärme aus der Destillation für die Pervaporation genutzt werden.

### Beispiel 1

### Trennung eines Gemisches mit Minimumazeotrop am Beispiel von Wasser und Tetrahydrofuran (THF)

Nach der Literaturstelle "Azeotropic data III", Advances in chemistry series 116, 1973, ACS, Washington, D.C., USA, liegt das Azeotrop von Wasser-THF bei Normaldruck bei 5,3 Gew.-% Wasser und siedet bei 64°C, gegenüber 66°C Siedetemperatur von reinem THF. Bei 7 bar verschiebt sich die azeotrope Zusammensetzung auf 12 Gew.-% Wasser. Es handelt sich um ein Minimumazeotrop mit einer ausgeprägten Druckabhängigkeit. Durch Pervaporation mit hydrophilen Membranen kann Wasser aus THF abgereichert werden.

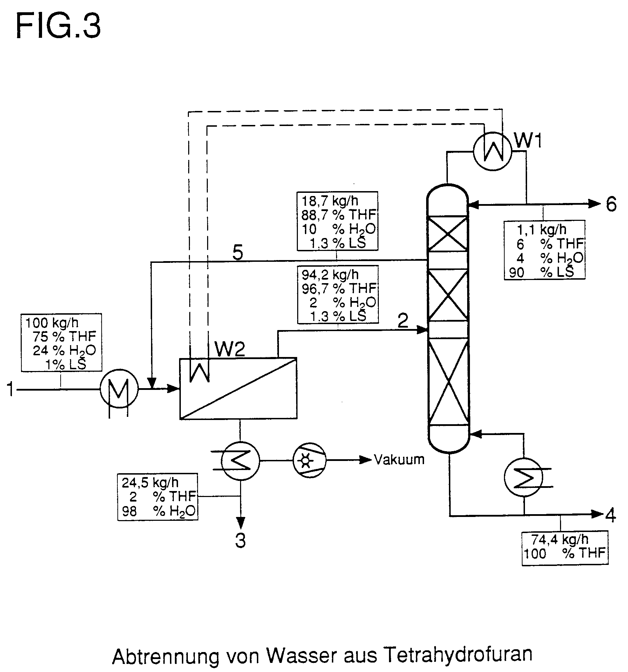

Figur 3 zeigt in Analogie zu Figur 1 die Anwendung des Verfahrens für die Trennung eines Flüssigkeitsgemischs mit 75 % THF, 24 % Wasser sowie 1 % eines Leichtsieders, der unterhalb des THF-Wasser Azeotrops siedet (Strom 1). Durch Pervaporation wird der Wassergehalt auf 2 % verringert (Strom 2). Es fällt ein Permeatstrom mit etwa 98 % Wasser und 2 % THF an (Strom 3). Im Sumpf der Destillationskolonne, die bei beispielsweise 5 bar Kopfdruck betrieben wird, wird THF rein gewonnen (Strom 4). Ein Wasser-THF Gemisch mit etwa azeotroper Zusammensetzung wird als flüssiger Seitenabzug im Verstärkungsteil entnommen (Strom 5) und wieder zurückgeführt. Der Leichtsieder wird oberhalb des Seitenabzugs auf ca. 90 % angereichert (Strom 6). Die Kondensationswärme der Destillationskolonne, die bei ca. 115°C anfällt, wird zur Beheizung der Pervaporation bei ca. 90°C genutzt (Wärmeverbund W1 -> W2, gestrichelte Linie).

Bei diesem Beispiel kommen alle vier genannten Vorteile der Erfindung zum Tragen.

### Beispiel 2

### Trennung eines Gemisches mit Maximumazeotrop am Beispiel von Chloroform und Aceton

Chloroform siedet unter Normaldruck bei 61,2°C, Aceton bei 56,5°C. Nach obiger Literaturstelle liegt das Azeotrop von Chloroform und Aceton bei 78,1 % Chloroform und siedet bei 64,4°C. Es handelt sich somit um ein Gemisch mit Maximumazeotrop.

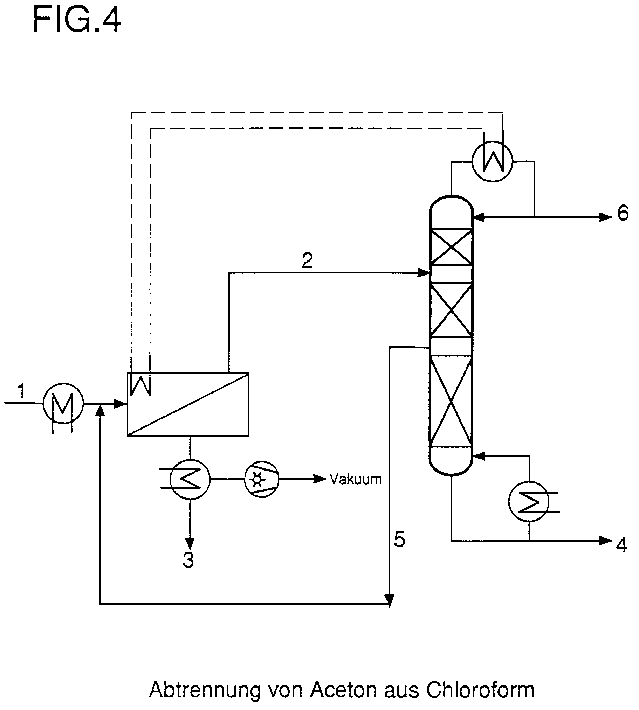

Figur 4 zeigt in Analogie zu Figur 2 die Anwendung des Verfahrens für die Trennung eines Flüssigkeitsgemischs mit z.B. 50 % Aceton und 50 % Chloroform (Strom 1). Durch Pervaporation wird der Acetongehalt auf eine Konzentration unterhalb der azeotropen Zusammensetzung, beispielsweise auf 10 % verringert (Strom 2). Am Kopf der Destillationskolonne, die bei Normaldruck betrieben wird, wird Chloroform rein gewonnen (Strom 6). Ein Aceton-Chloroform-Gemisch mit etwa azeotroper Zusammensetzung wird als Seitenabzug im Abtriebsteil entnommen (Strom 5) und wieder zurückgeführt. Die Schwersieder werden unterhalb des Seitenabzugs angereichert (Strom 4). Aceton reichert sich im Permeat an (Strom 3).

## Patentansprüche

1. Verfahren zur Trennung von Flüssigkeitsgemischen mit azeotropem Siedeverhalten durch Verschaltung von Pervaporation und Destillation, dadurch gekennzeichnet, daß
- in einem ersten Verfahrensschritt durch Pervaporation ein Gemisch soweit aufkonzentriert wird, daß die azeotrope Zusammensetzung überschritten wird, und
- in einem zweiten Verfahrensschritt die angereicherte Komponente bis zur geforderten Reinheit mittels einer Destillationseinheit aufdestilliert wird, und das dabei anfallende leichter oder schwerer siedende Gemisch der Pervaporation wieder zugeführt wird.

2. Verfahren nach Anspruch 1, dadurch gekennzeichnet, daß die Destillation bei erhöhtem Druck oder im Vakuum betrieben wird, um wegen der Druckabhängigkeit der azeotropen Zusammensetzung den zurückgeführten Mengenstrom zu verringern und/oder einen Wärmeverbund zwischen der Pervaporation und der Destillation zu ermöglichen.

3. Verfahren nach Anspruch 1, dadurch gekennzeichnet, daß das in der Destillation anfallende Gemisch als Seitenabzug entnommen wird, wenn weitere Komponenten abgetrennt werden sollen, die leichter flüchtig sind als ein zu trennendes Gemisch mit Minimumazeotrop bzw. schwerer flüchtig als ein Gemisch mit Maximumazeotrop.
